# EUROPEAN PATENT APPLICATION

(11) **EP 4 659 792 A1**
(43) Date of publication of application: **10.12.2025**
(21) Application number: 25180071.0
(22) Date of filing: 02.06.2025
(51) Int. Cl.: A61M 25/06, A61M 29/00

(54) **DELIVERY SYSTEM AND METHOD FOR PERCUTANEOUS INTRAVASCULAR PROCEDURES**

(30) Priority: 03.06.2024 US 202463655420 P; 28.05.2025 US 202519220589
(71) Applicant: Pacesetter, Inc., Sylmar, CA 91342 (US)
(72) Inventor: ARNAR, Bernhard, Minnetrista, MN (US)
(74) Representative: Barker Brettell Sweden AB

(57) **Abstract**

A delivery system (100) and method are described that include an access introducer sheath (102) and a delivery catheter (103). The access introducer sheath (102) defines a channel (124) through a body (126) thereof from a proximal end (120) to a distal end (122). The delivery catheter (103) is disposed within the channel (124) of the access introducer sheath (102), and defines a lumen (134) that extends along a central axis (114) of the delivery catheter (103). A distal end segment (136) of the delivery catheter (103) projects beyond the distal end (122) of the access introducer sheath (102). The lumen (134) is configured to receive a dilator (106) therein that projects beyond a distal end (112) of the delivery catheter (103) so that the distal end segment (136) of the delivery catheter (103) is disposed between a tip segment (116) of the dilator (106) and the distal end (122) of the access introducer sheath (102) along the central axis (114).

## Description

### TECHNICAL FIELD

Embodiments of the present disclosure relate generally to systems and methods related to percutaneous intravascular medical procedures, such as delivering implantable medical devices (IMDs).

### BACKGROUND

Delivery systems are used to gain access to blood vessels for cardiac catheterization, implanting IMDs on cardiac tissue, and other percutaneous intravascular medical procedures. The typical process involves first inserting a needle through the skin into the blood vessel. The needle has a small lumen through which a guidewire is inserted so that the guidewire extends through the skin and into the blood vessel. The needle is subsequently removed leaving the guidewire in place. A dilator with a lumen is then tracked over the guidewire. A tip of the dilator enlarges the opening in the skin and the puncture site of the blood vessel. The dilator is typically surrounded by an access introducer sheath as the dilator is tracked over the guidewire. The access introducer sheath follows the tip of the dilator through the puncture site into the blood vessel. The access introducer sheath has a channel that provides access to the blood vessel during the medical procedure. The dilator is subsequently removed from the access introducer sheath without displacing the access introducer sheath. One or more other components can be inserted through the channel of the access introducer sheath into the blood vessel for the procedure. The other components can include a catheter, an IMD, a lead for an IMD, a tool, and/or the like.

The access introducer sheath provides protection to patient tissues by limiting contact and forces exerted on the patient tissues during the medical procedure even as the other components are moved through the channel into and out of the blood vessel. The access introducer sheath maintains the skin opening site and the blood vessel puncture site in the open, patent state, and may be used to maintain hemostasis by limiting blood loss during the procedure.

There is a trend of the access introducer sheaths being formed with larger sizes in order to accommodate large payloads for catheter-based valve replacement procedures, leadless pacing device implants, and other cardiac procedures. The wall of the access introducer sheath has to be sufficiently thick and rigid to support the larger channel diameter and maintain the channel as open, unkinked, and unobstructed even when there is no component within the channel. For example, the wall has to be sufficiently strong to resist compressing, when there is no catheter or other device within the channel, due to the external pressure exerted on the outer surface of the access introducer sheath by the patient tissues and fluids. To increase the structural strength and rigidity, the access introducer sheath may be formed with strengthening materials, such as braided strands, coiled strands, and/or a discrete outer jacket.

In addition to requiring increased wall thickness and/or more complex structural design, larger access introducer sheaths may increase the risk for surgical complications. The surgical complications may include vessel dissections, perforations, inability to insert, and/or the like.

A need remains for a delivery system that avoids at least some of the issues with known percutaneous intravascular medical procedures that feature access introducer sheaths with relatively large internal channel diameters. For example, a need remains for delivery systems that increase efficiency of the procedure, reduce complexity, and reduce the risk of surgical complications compared to known delivery systems for percutaneous intravascular medical procedures.

### SUMMARY

In accordance with embodiments herein, a delivery system is provided that includes an access introducer sheath and a delivery catheter. The access introducer sheath has a proximal end and a distal end. The access introducer sheath defines a channel through a body of the access introducer sheath from the proximal end to the distal end. The delivery catheter is disposed within the channel of the access introducer sheath. The delivery catheter defines a lumen that extends along a central axis of the delivery catheter. A distal end segment of the delivery catheter projects beyond the distal end of the access introducer sheath. The lumen is configured to receive a dilator therein that projects beyond the distal end of the delivery catheter so that the distal end segment of the delivery catheter is disposed between a tip segment of the dilator and the distal end of the access introducer sheath along the central axis.

In an embodiment, the distal end of the delivery catheter may be tapered.

In an embodiment, the distal end of the access introducer sheath may be tapered.

In an embodiment, the delivery catheter may have a step that transitions from a broad segment of the delivery catheter to a narrow segment of the delivery catheter. The broad segment may be located between the distal end of the delivery catheter and the narrow segment. The access introducer sheath may surround the narrow segment, and the distal end of the access introducer sheath may abut against the step.

In an embodiment, a length of the access introducer sheath from the proximal end to the distal end may be less than 10 cm.

In an embodiment, the access introducer sheath may have a wall thickness of no greater than 0.254 mm.

In an embodiment, the access introducer sheath may have a mono-material composition.

In an embodiment, the delivery system may include a hub coupled to the access introducer sheath. The hub may include a housing and a hemostasis seal.

In an embodiment, the hub may be coupled to the proximal end of the access introducer sheath, and the hemostasis seal may engage an outer surface of the delivery catheter at a location spaced apart from the access introducer sheath to create a seal between the hub and the delivery catheter.

In an embodiment, the hemostasis seal may be an O-ring.

In an embodiment, the housing of the hub may include a base section that has a flat back side configured to be secured to skin of a patient to hold the access introducer sheath in a fixed position on the patient. The base section may include an adhesive on the flat back side for bonding to the skin of the patient.

In accordance with embodiments herein, a method of assembling a delivery system is provided. The method includes inserting a delivery catheter through a channel of an access introducer sheath. The channel of the access introducer sheath extends from a proximal end of the access introducer sheath to a distal end of the access introducer sheath. A distal end segment of the delivery catheter projects beyond the distal end of the access introducer sheath. The method includes loading a dilator through a lumen of the delivery catheter so that a tip segment of the dilator projects beyond the distal end segment of the delivery catheter. The distal end segment of the delivery catheter is disposed between the tip segment of the dilator and the distal end of the access introducer sheath along a central axis of the delivery catheter.

In an embodiment, the dilator, delivery catheter, and access introducer sheath may represent an entry assembly.

In an embodiment, the method may include coupling the access introducer sheath to a hub that comprises a housing and hemostasis seal, and sealing the hub to the delivery catheter via the hemostasis seal surrounding and engaging an outer surface of the delivery catheter at a location spaced apart from the access introducer sheath.

In an embodiment, the hemostasis seal may be an O-ring that is sized to seal around the outer surface of the delivery catheter as the delivery catheter is inserted through the channel of the access introducer sheath.

In an embodiment, the method may include forming the access introducer sheath to have at least one of: (i) a mono-material composition; (ii) a wall thickness of no greater than 0.254 mm; or (iii) a length that is less than 10 cm.

In accordance with embodiments herein, a delivery system is provided that includes an access introducer sheath, a hub, and a delivery catheter. The access introducer sheath has a proximal end and a distal end. The access introducer sheath defines a channel through a body of the access introducer sheath from the proximal end to the distal end. The hub is coupled to the access introducer sheath at or proximate to the proximal end. The hub includes a housing and a hemostasis seal. The delivery catheter is disposed within the channel of the access introducer sheath. The delivery catheter defines a lumen through a catheter body of the delivery catheter along a central axis of the delivery catheter. A distal end segment of the delivery catheter projects beyond the distal end of the access introducer sheath. The hemostasis seal engages an outer surface of the catheter body at a location spaced apart from the access introducer sheath to create a seal between the hub and the delivery catheter. The lumen of the delivery catheter is configured to receive a dilator therein that projects beyond the distal end segment of the delivery catheter so that the distal end segment of the delivery catheter is disposed between a tip segment of the dilator and the distal end of the access introducer sheath along the central axis.

### BRIEF DESCRIPTION OF THE DRAWINGS

Figure 1 illustrates a schematic cutaway view of a heart relative to an IMD.
Figure 2 is another schematic cutaway view of the heart showing a location of the bundle of His (e.g., His bundle) in the heart.
Figure 3 illustrates a delivery system formed in accordance with at least one embodiment.
Figure 4 illustrates a distal portion of the delivery system according to an embodiment.
Figure 5 is a cross-sectional view of the delivery system according to an embodiment.
Figure 6 is a side elevation view of a portion of the delivery system showing a dilator, catheter, and access introducer sheath according to an embodiment.
Figure 7 is a side elevation view of a portion of the delivery system showing the dilator, catheter, and access introducer sheath according to another embodiment.
Figure 8 is a side cross-sectional view of the portion of the delivery system shown in Figure 7.
Figure 9 illustrates a portion of the delivery system that includes a hub according to an embodiment.
Figure 10 illustrates the hub according to an embodiment with a portion of a housing removed to show a hemostasis seal.
Figure 11 is a cross-sectional view of the hub as shown in Figure 10.
Figure 12 is a flowchart of a method of assembling a delivery system according to an embodiment that can be used in a percutaneous intravascular medical procedure.
Figure 13 illustrates a block diagram of an exemplary IMD that is configured to be implanted into the patient in accordance with one or more embodiments herein.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

Embodiments may be implemented in connection with one or more implantable medical devices (IMDs). Non-limiting examples of IMDs include neurostimulator devices, implantable leadless monitoring and/or therapy devices, catheters, and/or alternative implantable medical devices. For example, the IMD may represent a cardiac monitoring device, pacemaker, cardioverter, cardiac rhythm management device, defibrillator, neurostimulator, leadless monitoring device, leadless pacemaker and the like. For example, the IMD may include one or more structural and/or functional aspects of the device(s) described in U.S. Patent 9,333,351 "Neurostimulation Method And System To Treat Apnea" and U.S. Patent 9,044,610 "System And Methods For Providing A Distributed Virtual Stimulation Cathode For Use With An Implantable Neurostimulation System."

Embodiments set forth herein include delivery systems for percutaneous intravascular medical procedures and methods of using the delivery systems for percutaneous intravascular medical procedures. Particular embodiments of the delivery system feature an entry assembly that includes an access introducer sheath, a dilator, and a delivery catheter. The access introducer sheath is integrated with the delivery catheter. The delivery system described herein has a different order of operation than conventional systems. For example, the dilator is introduced into the patient anatomy coupled to both the delivery catheter and the access introducer sheath. For example, the delivery catheter is located within the channel of the access introducer sheath, and the dilator extends through a lumen of the delivery catheter. After performing the function of enlarging the openings in the skin and the blood vessel, the dilator can be removed from the patient while both the delivery catheter and the access introducer sheath remain intact extending into the blood vessel. During the ensuing procedure, the delivery catheter may be moved through the channel of the access introducer sheath to navigate through the anatomy to a site of interest, such as a chamber of the heart. In this example, the delivery catheter is always located within the channel of the access introducer sheath, even when the dilator is present.

The delivery system described herein provides several beneficial technical effects. For example, the access introducer sheath may have a relatively thin wall thickness which beneficially limits the outer diameter of the sheath. Although the thin walls may sacrifice structural strength and rigidity, the delivery catheter provides internal structural support that maintains the sheath in the open, unkinked state. As described above, the delivery catheter may remain within the channel of the access introducer sheath during the procedure, so the sheath does not experience an empty channel. For a given channel diameter to accommodate a desired payload, the thin wall thickness permit a reduced sheath outer diameter. The smaller outer diameter, per the given channel diameter, beneficially reduces the risk of surgical complications by reducing the puncture size of the blood vessel. Furthermore, the access introducer sheath may have a relatively simple construction and composition which conserves part and/or manufacturing costs. The internal support provided by the delivery catheter may enable the access introducer sheath to be formed without braids, coils, outer jackets, and/or other integrated reinforcements.

Another technical effect of the delivery system described herein may be a more efficient procedure that has a reduced number of steps than the known procedure. For example, the delivery system and method described herein do not insert a delivery catheter into the access introducer sheath after removing the dilator from the access introducer sheath. This step is avoided because the delivery catheter according to one or more the embodiments described herein is already coupled within the channel of the sheath when the sheath is advanced through the skin of the patient into the blood vessel. The delivery system may also avoid the use of a valve bypass tool, which saves costs and time. For example, a valve bypass tool may be used in known delivery systems to permit a catheter, IMD, and/or other delivery tool to bypass a hemostasis valve and enter the channel of the access introducer sheath after the dilator is removed from the sheath. In an embodiment described herein, the delivery catheter is pre-loaded into the channel of the sheath prior to entering the patient anatomy, so there is no need to pierce the hemostasis seal using a special valve bypass tool.

In an example application, the delivery system may be used to deliver an IMD, or a portion of the IMD, to cardiac tissue of the patient. The portion of the IMD may be a lead (e.g., a pacing lead). The IMD may be a pacemaker that includes the lead, a leadless pacemaker, or the like. When implanted at a target implant site, the lead and/or leadless pacemaker may deliver electrical stimulation therapy to the cardiac tissue and/or monitor an evoked response to the electrical stimulation therapy. The electrical stimulation therapy may include pacing pulses, defibrillation shock pulses, and/or the like. The IMD and/or the lead may be delivered to the implant site through the delivery catheter of the delivery system. For example, the lead may be advanced through a lumen of the delivery catheter. In another example, a leadless pacemaker or other IMD may be pushed by a rod or other instrument through the lumen of the delivery catheter to the implant site. Once the IMD and/or lead is successfully implanted at an appropriate location of the heart, the delivery catheter and the access introducer sheath may be removed together from the patient while the IMD and/or lead remains implanted.

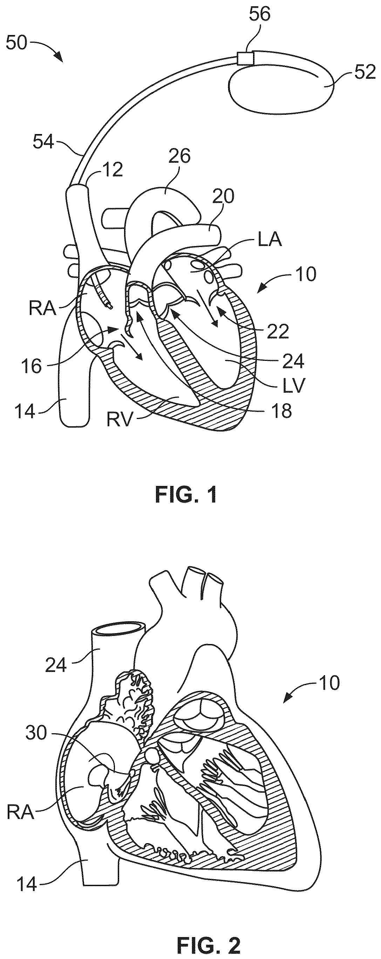

Figure 1 illustrates a schematic cutaway view of a heart 10 relative to an IMD 50. The IMD 50 includes a lead 54 that may be delivered to the heart 10 via the delivery system described herein. The heart 10 includes a right atrium RA, a right ventricle RV, a left atrium LA, and a left ventricle LV. During normal operation of the heart 10, deoxygenated blood from the body is returned to the right atrium RA from the superior vena cava 12 and inferior vena cava 14. The right atrium RA pumps the blood through the atrioventricular or tricuspid valve 16 to the right ventricle RV, which then pumps the blood through the pulmonary valve 18 and the pulmonary artery 20 to the lungs for reoxygenation and removal of carbon dioxide. The newly oxygenated blood from the lungs is transported to the left atrium LA, which pumps the blood through the mitral valve 22 to the left ventricle LV. The left ventricle LV pumps the blood through the aortic valve 24 and the aorta 26 throughout the body.

Figure 2 is another schematic cutaway view of the heart 10 showing a location of the bundle of His 30 (e.g., His bundle) in the heart. The His bundle 30 consists of fast-conducting muscle fibers that begin at the atrioventricular node in the right atrium and pass to the interventricular septum. The His bundle 30 divides in the septum into a right branch that travels along the right side of the septum and supplies excitation to the right ventricle, and a pair of left branches that travel along the left side of the septum and supply excitation to the left ventricle. The fibers in the branches terminate in an extensive network of Purkinje fibers which distribute excitation pulses to the layer of cells beneath the endocardium.

Returning to Figure 1, the IMD 50 includes a pulse generator 52 that is operably coupled to the lead 54 through a lead adaptor 56. A proximal end of the lead 54 may be coupled to the pulse generator 52 after the lead 54 is delivered to the implant site by the delivery system described herein. The lead adaptor 56 is configured to receive a lead connector (not shown) of the lead 54. Although the IMD 50 includes only one lead in Figure 1, the IMD 50 may include multiple leads in other embodiments. The lead 54 may be designed to penetrate the endocardium in contact with His bundle 30. The lead 54 may enter the vascular system through one of several possible vascular access sites. For example, the lead 54 may enter through the femoral artery/vein. The lead 54 may extend through the superior vena cava 12 to the right atrium RA.

In Figure 1, the IMD 50 may be a cardiac pacemaker. In other embodiments, however, the IMD 50 may include an ICD, a CRT-D, an ICD coupled with a pacemaker, and the like. The IMD 50 may be a dual-chamber stimulation device capable of treating both fast and slow arrhythmias with stimulation therapy, including cardioversion, defibrillation, and pacing stimulation, as well as capable of detecting heart failure, evaluating its severity, tracking the progression thereof, and controlling the delivery of therapy and warnings in response thereto. The IMD 50 may be controlled to sense atrial and ventricular waveforms of interest, discriminate between two or more ventricular waveforms of interest, deliver stimulus pulses or shocks, and inhibit application of a stimulation pulse to a heart based on the discrimination between the waveforms of interest and the like.

Although not shown, the IMD 50 may wirelessly communicate with an external device. The external device may be used by a physician or other technician to select and/or modify therapy parameters to be implemented by the IMD 50.

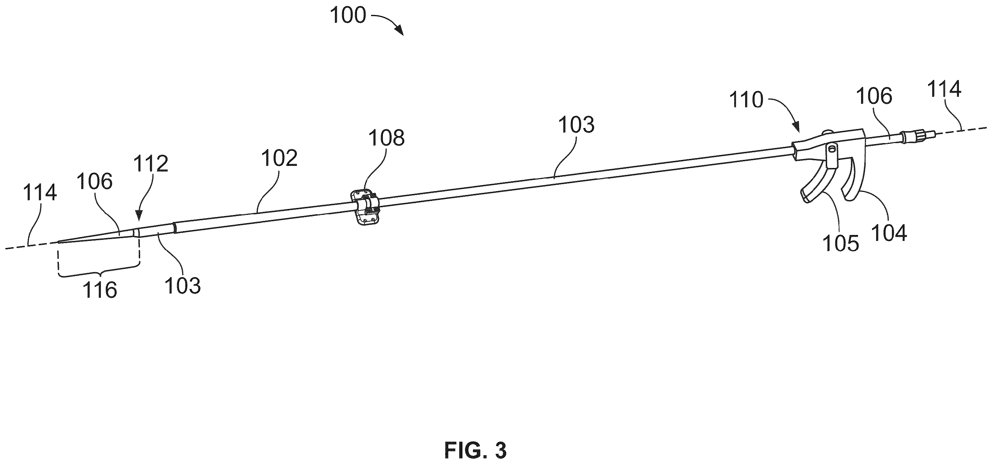

Figure 3 illustrates a delivery system 100 formed in accordance with at least one embodiment. The delivery system 100 may be used for percutaneous intravascular medical procedures. For example, the delivery system 100 may be used to deliver an IMD, or a lead thereof, to a target implant site, cardiac catheterization, and/or the like. The target implant site optionally may be within a chamber of the heart. For example, the delivery system 100 may be used to deliver the lead 54 of the IMD 50 shown in Figure 2. In other applications, the delivery system 100 can be used for percutaneous medical procedures that are not intravascular, such as implanting subcutaneous IMDs outside of the heart.

The delivery system 100 includes an access introducer sheath 102, a delivery catheter 103, a handle 104, a dilator (or obturator) 106, and a hub 108. The delivery system 100 may include more components than shown in Figure 3, such as a fluid flushing assembly, and electrical connector, and/or the like. The handle 104 may be coupled to a proximal end 110 of the delivery catheter 103 (also referred to herein as catheter 103). The handle 104 may include a lever 105 that is manually manipulated by an operator to cause the catheter 103 to bend. The operator may use the handle and lever to steer the catheter 103 through the tortuous vascular system of the patient to a target implant site.

The catheter 103 is a hollow shaft or tube (e.g., a guide tube). The catheter 103 may be used to deliver an IMD or lead, such as by providing a guide path along which the IMD or lead can be advanced to the target implant site. The catheter 103 may be steered to position a distal end 112 of the catheter 103 proximate to the target implant site. The catheter 103 may include a plurality of sheath segments along its length that are bendable relative to other sheath segments. The catheter 103 may be maneuverable and have sufficient columnar strength for navigating through the tortuous vascular system. The catheter 103 may also have sufficient kink-resistance so as to bend smoothly. The catheter 103 is elongated and extends lengthwise along a central axis 114 of the catheter 103 from the proximal end 110 to the distal end 112. The central axis 114 may extend along a curved path that changes as the catheter 103 is flexed, bent, twisted, or otherwise manipulated.

The access introducer sheath 102 surrounds a section of the catheter 103. The hub 108 is secured to the access introducer sheath 102. The catheter 103 is movable relative to the access introducer sheath 102 and the hub 108. In Figure 3, the catheter 103 surrounds the dilator 106. The dilator 106 is used to enlarge an opening in the skin and/or enlarge a puncture opening in a blood vessel (e.g., a vein or an artery). The dilator 106 has a tip segment 116 at a distal end that projects beyond the distal end 112 of the catheter 103. The tip segment 116 is tapered. The tip segment 116 may be cone-shaped (e.g., conical). In another example, the tip segment 116 may be wedge-shaped. The dilator 106 can be removed from the catheter 103 after enlarging the opening(s) by pulling the dilator 106 in a proximal direction along the central axis 114 out of the proximal end 110 of the catheter 103 and beyond the handle 104.

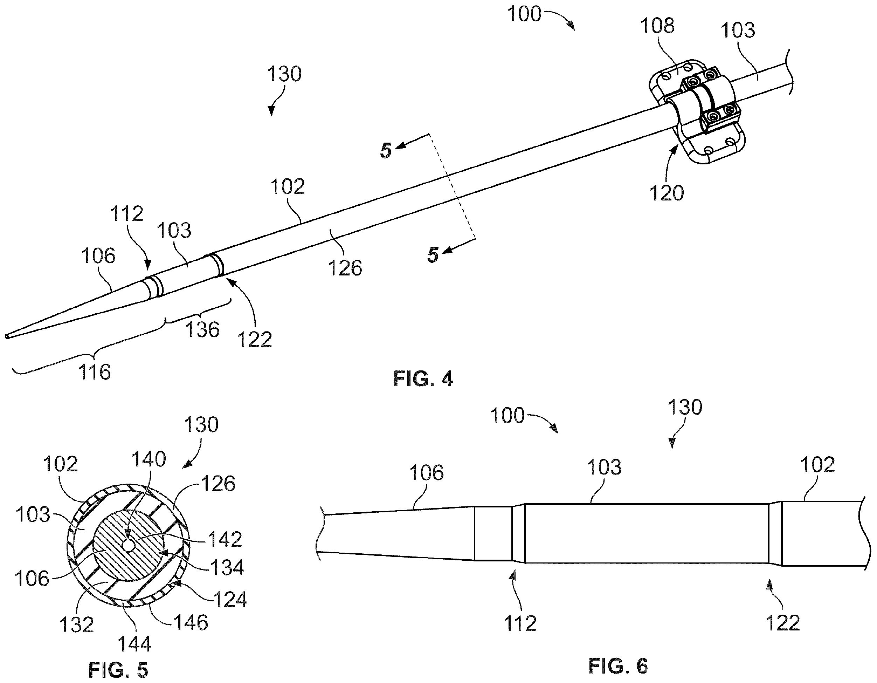

Figure 4 illustrates a distal portion of the delivery system 100 according to an embodiment. The distal portion shows the entire length of the access introducer sheath 102 and the hub 108. The access introducer sheath 102 has a proximal end 120 and a distal end 122. In Figure 4, the access introducer sheath 102 surrounds a portion of the catheter 103, which surrounds at least a portion of the dilator 106. When assembled as shown in Figures 3 and 4, the access introducer sheath 102, catheter 103, and dilator 106 define or represent an entry assembly 130.

Figure 5 is a cross-sectional view of the delivery system 100 according to an embodiment. The cross-section is taken along the line 5-5 in Figure 4, and the view is directed in a distal direction along the central axis 114. The cross-section line 5-5 extends through the entry assembly 130, including the access introducer sheath 102, the catheter 103, and the dilator 106.

With reference to both Figures 4 and 5, the access introducer sheath 102 defines a channel 124 that extends through a body 126 of the access introducer sheath 102. The channel 124 extends the length of the access introducer sheath 102 from the proximal end 120 to the distal end 122. The channel 124 is open at both ends 120, 122. The catheter 103 is disposed (e.g., located) within the channel 124 of the access introducer sheath 102. The catheter 103 is longer than the access introducer sheath 102. For example, the catheter 103 may project beyond both the proximal and distal ends 120, 122. The portion of the catheter 103 that projects beyond the distal end 122 is referred to herein as a distal end segment 136 of the catheter 103. The distal end segment 136 may be exposed to the surrounding environment. The catheter 103 has a body 132 (referred to herein as a catheter body). The catheter 103 defines a lumen 134 through the catheter body 132. The lumen 134 extends lengthwise along the central axis 114 from the proximal end 110 to the distal end 112. The lumen 134 may be open at both ends 110, 112.

The lumen 134 is sized and shaped to receive the dilator 106 therein. For example, a cross-sectional shape of the lumen 134 may correspond to a cross-sectional shape of the outer surface of the dilator 106. In the illustrated example, the lumen shape is circular, and the outer surface of the dilator 106 is circular (e.g., cylindrical). Furthermore, a cross-sectional dimension (e.g., diameter) of the lumen 134 may be only slightly larger than the cross-sectional dimension of the outer surface (e.g., the outer diameter) of the dilator 106. For example, the dilator 106 may fit within the lumen 134 of the catheter 103 with a relatively snug fit while permitting movement of the dilator 106 through the catheter 103 without undue force to overcome friction. The dilator 106 has the tip segment 116 that projects beyond the distal end 112 of the catheter 103. When assembled to form the entry assembly 130, the distal end segment 136 of the catheter 103 is axially disposed (e.g., along the central axis 114) between the tip segment 116 of the dilator 106 and the distal end 122 of the access introducer sheath 102. The dilator 106 may define a small aperture (e.g., lumen) 140 through a body 142 (referred to herein as dilator body) of the dilator 106. The aperture 140 may extend the length of the dilator 106 along the central axis 114. The aperture 140 may be sized to receive a guidewire therethrough.

The delivery system 100 may have the entry assembly 130 as shown in Figures 3 through 5 during an early step in a percutaneous medical procedure. For example, an initial step may be to gain access to a blood vessel by inserting a needle into the skin and then into the blood vessel. In an example, the blood vessel may be a femoral artery or vein. A guidewire may then be inserted into a lumen of the needle and advanced through the openings in the skin and the blood vessel into the blood vessel. The needle may be removed while the guidewire remains extending through the skin into the blood vessel. Once the needle is removed, the entry assembly 130 of the delivery system 100 may be advanced over the guidewire. For example, a proximal end of the guidewire may be inserted into the aperture 140 of the dilator 106 at the distal end of the tip segment 116.

The entry assembly 130 is then advanced into the patient along the length of the guidewire. For example, the tapered tip segment 116 of the dilator 106 first extends through the opening in the skin to enlarge the access opening. The distal end segment 136 of the catheter 103 penetrates the skin through the access opening following the tip segment 116 as the delivery system 100 is advanced in a loading direction following the path of the guidewire. The access introducer sheath 102 enters the patient through the access opening in the skin, distal end 122 first, by continuing to advance the delivery system 100 in the loading direction. In an example, the entire delivery system 100 shown in Figure 3 may be advanced in the loading direction. For example, the positioning of the components of the delivery system 100 may remain constant during this process of moving the delivery system 100 along the guidewire into the patient anatomy.

After penetrating the skin, the tip segment 116 of the dilator 106 may follow the guidewire into the blood vessel. The tapered tip segment 116 may gradually enlarge a puncture site of the blood vessel. The puncture site is gradually enlarged to enable the blood vessel to permit access to the catheter 103 and the access introducer sheath 102 without trauma or with only limited trauma. For example, the tapered tip segment 116 may gradually enlarge the puncture site to avoid (or reduce the risk of) tearing the thin walls of the blood vessel when the catheter 103 and/or access introducer sheath 102 are advanced through the puncture site. Similar to the opening in the skin, the distal end segment 136 of the catheter 103 and then the distal end 122 of the access introducer sheath 102 may follow the tip segment 116 of the dilator 106 into the blood vessel through the puncture site.

The delivery system 100 may be advanced as a unit until the access introducer sheath 102 extends through both of the openings in the skin and the blood vessel. At this position, the hub 108 may be located external of the patient proximate to the transdermal puncture site. The hub 108 may then be secured to the skin of the patient to hold the access introducer sheath 102 in place during the ensuing medical procedure. The hub 108 may be secured to the skin via sutures, adhesive, and/or the like. After securing the hub 108 and the access introducer sheath 102, an operator may remove the dilator 106 from the lumen 134 of the catheter 103 without removing the catheter 103. For example, the operator may pull the dilator 106 in a proximal direction to extract the dilator 106 from the patient. Then, the operator may use the catheter 103 to continue to the procedure. For example, the operator may manipulate the catheter 103 to steer the catheter 103 through the vascular system of the patient to the target implant site. The access introducer sheath 102 and the hub 108 may remain fixed in place while the catheter 103 is moved. For example, the catheter 103 may be moved axially to slide through the channel 124 of the access introducer sheath 102.

The procedure may involve loading an IMD and/or a lead through the now empty lumen 134 of the catheter 103 for delivery to the implant site. Optionally, the procedure may involve loading one or more tools through the lumen 134 of the catheter 103. Once the procedure is completed, the operator may release the hub 108 from the skin and then extract the remaining components of the delivery system 100 from the patient as a unit. For example, the operator may pull the delivery system 100 to concurrently back out both the access introducer sheath 102 and the catheter 103 from the patient.

The delivery system 100 differs from known delivery system in which the dilators and catheters are discrete components that are not used together. For example, the convention procedure uses a dilator with an access introducer sheath to install the access introducer sheath. Then, the dilator is removed from the access introducer sheath, and a catheter (e.g., delivery device) is loaded into the access introducer sheath in place of the dilator. The delivery system 100 described herein integrates the dilator 106 with the catheter 103 and the access introducer sheath 102. For example, all three components couple (e.g., nest) together to form the entry assembly 130. Known delivery systems do not load a dilator through a lumen of a catheter (e.g., delivery device). The catheter 103 of the delivery system 100 enters the patient during the same step of the procedure as the dilator 106 and the access introducer sheath 102. This modification improves the efficiency of the medical procedure by eliminating the discrete step of inserting the catheter into the access introducer sheath after removing the dilator. A secondary benefit is that the delivery system 100 may operate without a valve bypass tool and without a bulky hemostasis valve. The hub 108 may provide a suitable hemostasis seal.

As shown in Figure 5, the portion of the catheter 103 within the channel 124 of the access introducer sheath 102 provides structural support to the sheath 102. For example, the catheter 103 prohibits the wall of the access introducer sheath 102 from deflecting (e.g., compressing, kinking, etc.), which maintains the channel 124 as open and patent. As described above, the catheter 103 may remain within the channel 124 during the entire time that the access introducer sheath 102 is used during the medical procedure. The wall of the access introducer sheath 102 may not require sufficient strength and rigidity to withstand forces exerted on the sheath 102 during the procedure while the channel 124 is empty, because the channel 124 may not be empty during the procedure. In an example, the body 126 of the access introducer sheath 102 may be formed with a thin wall thickness. The body 126 has a wall 144 that has a thickness radially extending from an inner surface (defining the channel 124) to an outer surface 146 of the access introducer sheath 102. The wall thickness can be thin to beneficially limit the outer diameter of the access introducer sheath 102 (e.g., which defines the diameter of the entry assembly 130). Limiting the outer diameter may reduce the risk of trauma to patient tissues, such as the blood vessel walls. In an example, the thickness of the wall 144 may be no greater than 0.254 mm (0.01 in.) For example, the thickness may be approximately 0.127 mm (0.005 in.), and the outer diameter of the access introducer sheath 102 may be approximately 8.331 mm (0.328 in. or 25 French (F)). The channel 124 may be relatively large to accommodate a large payload.

Furthermore, the access introducer sheath 102 may have a relatively simple construction. In an example, the body 126 of the access introducer sheath 102 may have a mono-material composition, such that the entire sheath 102 is formed by a single material composition. In an example, the mono-material is a thermoplastic. The access introducer sheath 102 may be formed by extruding the mono-material. The access introducer sheath 102 may omit reinforcing elements that are present in some known access introducer sheaths for enhancing the material strength. As a result, the access introducer sheath 102 may be relatively cheaper to manufacture. In another embodiment, the access introducer sheath 102 may include multiple different materials and/or reinforcing elements.

The length of the access introducer sheath 102 may be selected so that the access introducer sheath 102 is sufficiently long to extend from the hub 108 secured to the skin of the patient near the access opening in the skin into the access opening and into the puncture site of the blood vessel. The access introducer sheath 102 may be shorter than some known access introducer sheaths. For example, the length of the access introducer sheath 102 from the proximal end 120 to the distal end 122 may be less than 10 cm. Optionally, the length may be 5 cm or less, depending on the proximity of the blood vessel to the skin access opening and the ability to secure the hub 108 near the skin access opening.

Figure 6 is a side elevation view of a portion of the delivery system 100 showing the dilator 106, catheter 103, and access introducer sheath 102 according to an embodiment. The components are in the entry assembly 130 configuration and poised for entry through the access opening of a patient's skin, as shown in Figures 3 and 4. In an example, the distal end 112 of the catheter 103 is tapered. For example, the diameter of the catheter 103 gradually reduces with increasing distance in the distal direction. The tapered distal end 112 may avoid or at least limit trauma to patient tissue. For example, the tapered distal end 112 provides a gradual transition from the diameter of the dilator 106 to the diameter of the catheter 103, which reduces the likelihood of snagging and/or tearing tissue. The tapered distal end 112 of the catheter 103 may fit tightly on the dilator 106 so as to reduce trauma to the skin and vessel walls when advancing.

In an example, the distal end 122 of the access introducer sheath 102 is tapered. For example, both the distal ends 112, 122 of the catheter 103 and the access introducer sheath 102 may be tapered. The tapered distal end 122 may avoid or at least limit trauma to the patient tissue for a similar reason as the tapered distal end 112. For example, the tapered distal end 112 provides a gradual transition from the diameter of the catheter 103 to the diameter of the access introducer sheath 102. The tapered distal end 122 may fit tightly on the catheter 103 so as to reduce trauma to the skin and vessel walls when advancing.

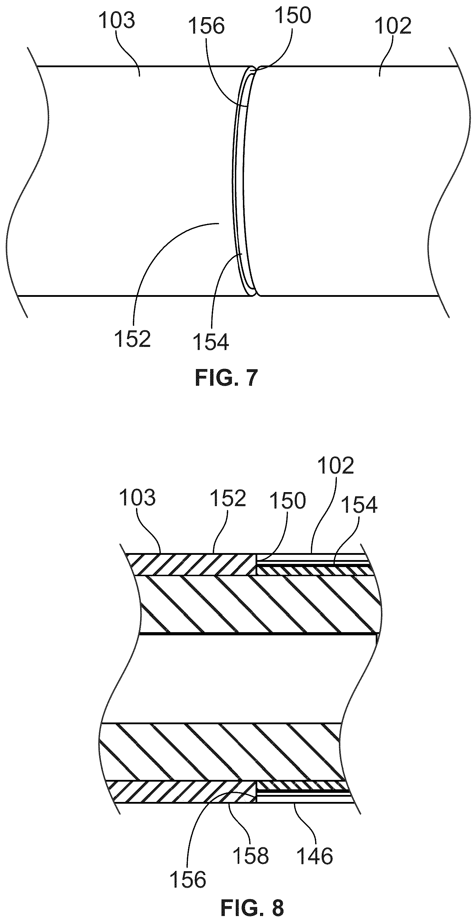

Figure 7 is a side elevation view of a portion of the delivery system 100 showing the dilator 106, catheter 103, and access introducer sheath 102 according to another embodiment. Figure 8 is a side cross-sectional view of the portion of the delivery system 100 shown in Figure 7. The catheter 103 has the same tapered distal end 112 that is shown in Figure 6, but the distal end 122 of the access introducer sheath 102 is not tapered. In an example, the catheter body 132 of the catheter 103 includes an annular step 150 that transitions from a broad segment 152 of the catheter body 132 to a narrow segment 154 of the catheter body 132. The broad segment 152 has a slightly larger outer diameter than the narrow segment 154. The broad segment 152 is distal of the narrow segment 154, such that it is located between the distal end 112 of the catheter 103 and the narrow segment 154. The step 150 may be a blunt, right angle transition. The access introducer sheath 102 surrounds the narrow segment 154, and does not surround the broad segment 152. For example, the broad segment 152 may define the distal end segment 136 of the catheter 103 that projects beyond the sheath 102. The distal end 122 of the access introducer sheath 102 may abut against the step 150. For example, the distal end 122 may have a blunt and/or square-cut edge 156 instead of being tapered.

The blunt edge 156 may abut the step 150 when the access introducer sheath 102 attains a fully loaded position on the catheter 103. In Figure 7, the blunt edge 156 is spaced apart from the step 150 because the access introducer sheath 102 is not in the fully loaded position on the catheter 103. Figure 8 shows the blunt edge 156 in contact within the step 150. The access introducer sheath 102 is in the fully loaded position on the catheter 103 in Figure 8. In an example, the outer surface 146 of the access introducer sheath 102 may be approximately flush with an outer surface 158 of the catheter 103 along the broad segment 152. For example, the radial dimension of the annular step 150 may be approximately the same as the wall thickness of the access introducer sheath 102. The flush coupling may result in a smooth transition between the catheter 103 and the access introducer sheath 102, reducing trauma to the skin and the blood vessel when insetting the delivery system 100 into the patient. For example, the flush coupling may reduce the risk of snagging on patient tissue at the transition.

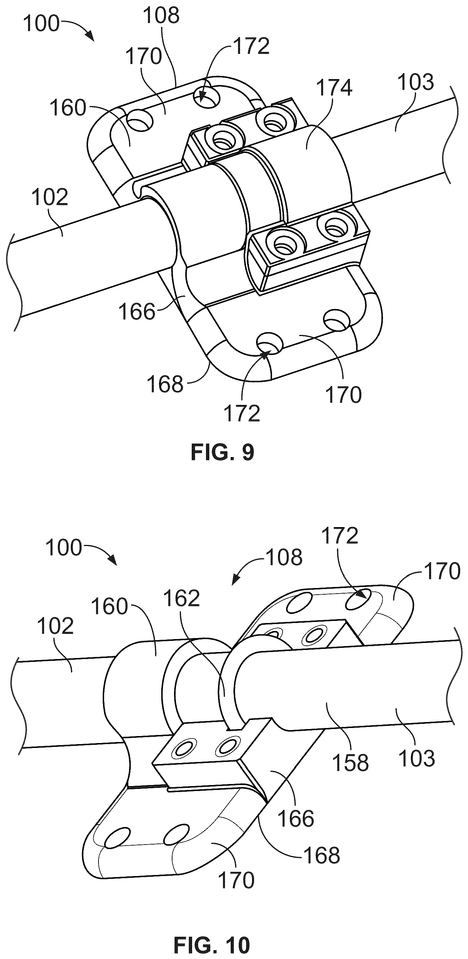

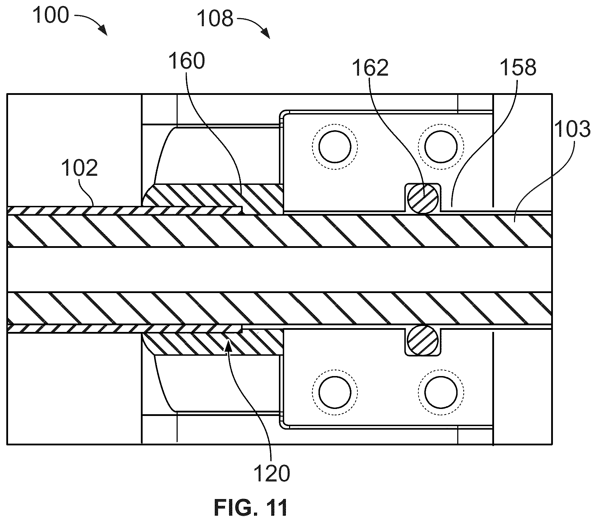

Figure 9 illustrates a portion of the delivery system 100 that includes the hub 108 according to an embodiment. The hub 108 includes a housing 160 and a hemostasis seal 162. The hub 108 may extend along a proximal transition area between the access introducer sheath 102 and the catheter 103. The proximal transition area includes the proximal end 120 (shown in Figure 11) of the access introducer sheath 102 and the portion of the catheter 103 projecting from the proximal end 120. The housing 160 may surround and physically contact both the access introducer sheath 102 and the catheter 103 along the proximal transition area.

The housing 160 holds the hemostasis seal 162. Figure 10 illustrates the hub 108 according to an embodiment with a portion of the housing 160 removed to show the hemostasis seal 162. Figure 11 is a cross-sectional view of the hub 108 as shown in Figure 10. The portion of the housing 160 omitted in Figures 10 and 11 is referred to herein as a closure section 174 (shown in Figure 9). The hemostasis seal 162 is designed to block blood from leaking out of the access introducer sheath 102 when the access introducer seal 102 extends into the patient. In an example, the hemostasis seal 162 seals to the outer surface 158 of the catheter 103 to create a seal between the hub 108 and the catheter 103. The hemostasis seal 162 may be spaced apart from the access introducer sheath 102. For example, the seal 162 may be located proximal of the proximal end 120 of the access introducer sheath 102. The seal 162 may be a wiper seal that is contained within the housing 160. In an example, the seal 162 seals to the outer surface 158 of the catheter 103 via an interference (e.g., friction) fit. The seal 162 may be annular. For example, the seal 162 may be an O-ring. The O-ring may engage (e.g., grip) the catheter 103 via an interference fit to block (e.g., seal) a leak path between the housing 160 and the catheter 103.

The housing 160 of the hub 108 may include a base section 166 that has a flat back side 168 configured to be secured to the skin of the patient to hold the access introducer sheath 102 in a fixed position on the patient. The housing 160 may also have the closure section 174 that secures to the base section 166 to cause the hub 108 to grip the access introducer sheath 102 and the catheter 103. The closure section 174 may be coupled to the base section 166 via fasteners, as shown, or by a latch or another coupling mechanism. The flat back side 168 may permit the housing 160 to lay flat on the patient with limited risk of rotation relative to the patient skin. The housing 160 may also have a relatively low profile to reduce the risk of snagging on tools, clothes, or the like, during the medical procedure. The base section 166 may include wings 170, at least one flange, or the like, that define the flat back side 168. The base section 166 may include suture holes 172 designed to allow a physician to suture the hub 108 to the patient's skin for securing the hub 108 in place. The base section 166 may include an adhesive on the flat back side 168 for bonding to the skin of the patient. For example, a physician may peel a cover layer or film from the flat back side 168 to expose an adhesive layer on the flat back side 168 before pressing the housing 160 against the skin to bond.

As shown in Figures 9 through 11, the hub 108 may be a relatively simple and non-complex structure that is relatively small and low profile. The hub 108 may provide sufficient hemostasis sealing without using a conventional seal that is bulkier and more complex. For example, the hub 108 may be coupled to the delivery system 100 when the catheter 103 is already within the channel 124 of the access introducer sheath 102, so the hub 108 can be assembled directly to the catheter 103 and the access introducer sheath 102. The hub 108 does not have to provide a hemostasis seal when the access introducer sheath 102 is unoccupied (e.g., empty), so the hub 108 may have a relatively simple design and compact size.

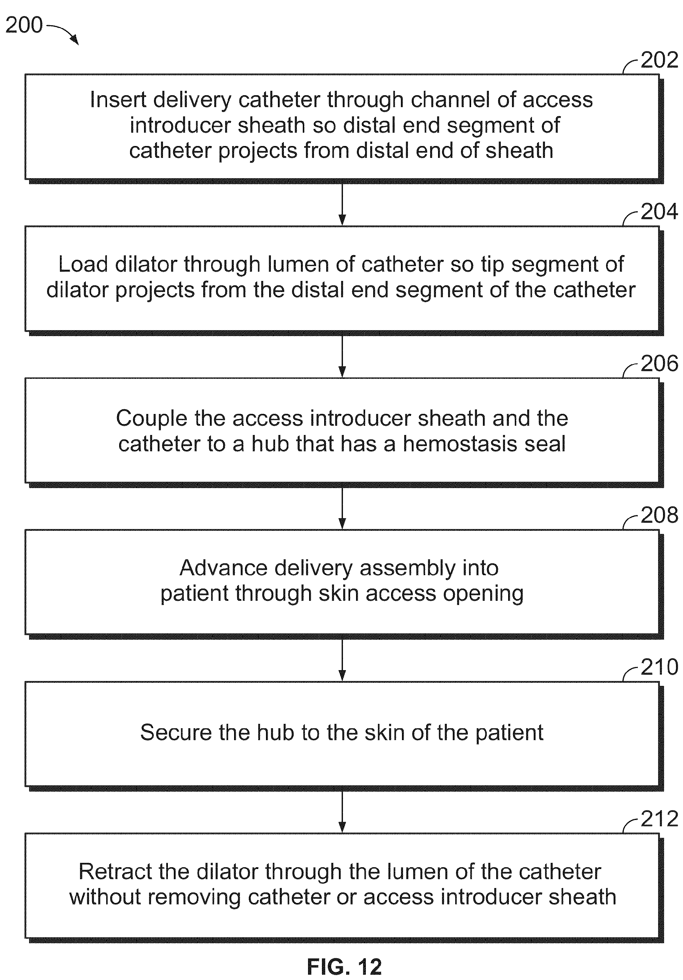

Figure 12 is a flowchart 200 of a method of assembling a delivery system 100 according to an embodiment. The method may assemble and use the delivery system 100 shown and described in Figures 1 through 11. For example, the initial steps of the method may assemble the delivery system 100. Subsequent steps may involve the use of the delivery system 100 in the percutaneous intravascular medical procedure. In different embodiments, the method may include different steps not shown in Figure 12, may omit one or more of the steps shown in Figure 12, and/or may have a different order of the steps than shown in Figure 12.

At step 202, a delivery catheter 103 is inserted through a channel 124 of an access introducer sheath 102 so that a distal end segment 136 of the catheter 103 projects beyond a distal end 122 of the access introducer sheath 102. The method optionally may include forming the access introducer sheath 102. The access introducer sheath 102 may be formed to have at least one of: (i) a mono-material composition; (ii) a wall thickness of no greater than 0.254 mm; and/or (iii) a length that is less than 10 cm.

At step 204, a dilator 106 is loaded through a lumen 134 of the catheter 103 so that a tip segment 116 of the dilator 106 projects beyond the distal end segment 136 of the catheter 103. Steps 202 and 204 may be performed in any chronological order. The distal end segment 136 of the delivery catheter 103 is disposed between the tip segment 116 of the dilator 106 and the distal end 122 of the access introducer sheath 102 along a central axis 114 of the catheter 103.

At step 206, the access introducer sheath 102 and the catheter 103 are coupled to a hub 108 that includes a housing 160 and a hemostasis seal 162. The dilator 106, catheter 103, access introducer sheath 102, and hub 108 define or represent an entry assembly 130. The hemostasis seal 162 of the hub 108 seals to the catheter 103 by surrounding and engaging an outer surface 158 of the catheter 103 at a location spaced apart from the sheath 102. The hemostasis seal 162 may be an O-ring that is sized to seal around the outer surface 158 of the catheter 103 as the catheter 103 is inserted through the channel 124 of the access introducer sheath 102.

At step 208, the entry assembly 130 is advanced into a patient through an access opening in the skin. The entry assembly 130 is advanced in a loading direction with the tip segment 116 of the dilator 106 entering the patient first, followed by the distal end segment 136 of the catheter 103 and then the distal end 122 of the sheath 102. The dilator 106, catheter 103, and sheath 102 may be advanced through a puncture opening in a blood vessel. At step 210, the hub 108 is secured to the skin of the patient to secure the sheath 102 in a fixed position during the procedure. In an example, an adhesive on a flat back side 168 of the housing 160 is used to bond the housing 160 to the patient's skin. At step 212, the dilator 106 is retracted through the lumen 134 of the catheter 103 without removing the sheath 102 or the catheter 103 from the patient. After removing the dilator 106, an IMD, a lead of an IMD, a tool, or the like may be inserted into and advanced through the lumen 134 of the catheter 103 to access the vascular system of the patient for the medical procedure.

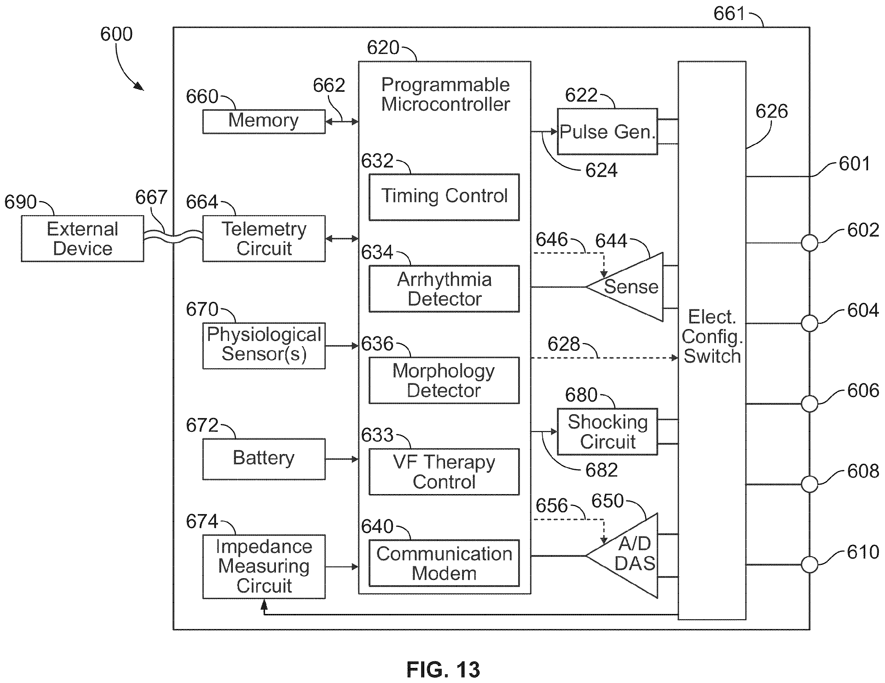

Figure 13 illustrates a block diagram of an exemplary IMD that is configured to be implanted into the patient in accordance with embodiments herein. The IMD 600, or a portion thereof, may be implanted using the delivery system 100. The IMD 600 may treat both fast and slow arrhythmias with stimulation therapy, including cardioversion, pacing stimulation, an implantable cardioverter defibrillator, suspend tachycardia detection, tachyarrhythmia therapy, and/or the like.

The IMD 600 has a housing 661 to hold the electronic/computing components. The housing 661 (which is often referred to as the "can," "case," "encasing," or "case electrode") may be programmably selected to act as the return electrode for certain stimulus modes. The housing 661 further includes a connector (not shown) with a plurality of terminals 601, 602, 604, 606, 608, and 610. The terminals may be connected to one or more leads that are located in various locations within and about the heart. Each lead may have one or more electrodes. The type and location of each electrode may vary. For example, the electrodes may include various combinations of ring, tip, coil, shocking electrodes, and the like.

The IMD 600 includes a programmable microcontroller 620 that controls various operations of the IMD 600, including cardiac monitoring and stimulation therapy. The microcontroller 620 includes a microprocessor (or equivalent control circuitry), one or more processors, RAM and/or ROM memory, logic and timing circuitry, state machine circuitry, and I/O circuitry. The IMD 600 further includes a pulse generator 622 that generates stimulation pulses for connecting the desired electrodes to the appropriate I/O circuits, thereby facilitating electrode programmability. The switch 626 is controlled by a control signal 628 from the microcontroller 620.

Optionally, the IMD 600 may include multiple pulse generators, similar to the pulse generator 622, where each pulse generator is coupled to one or more leads/electrodes and controlled by the microcontroller 620 to deliver select stimulus pulse(s) to the corresponding one or more electrodes. The IMD 600 includes sensing circuit 644 selectively coupled to one or more electrodes that perform sensing operations, through the switch 626 to detect the presence of cardiac activity in the chamber of the heart. The output of the sensing circuit 644 is connected to the microcontroller 620 which, in turn, triggers, or inhibits the pulse generator 622 in response to the absence or presence of cardiac activity. The sensing circuit 644 receives a control signal 646 from the microcontroller 620 for purposes of controlling the gain, threshold, polarization charge removal circuitry (not shown), and the timing of any blocking circuitry (not shown) coupled to the inputs of the sensing circuit 624.

In the example of Figure 13, the sensing circuit 644 is illustrated. Optionally, the IMD 600 may include multiple sensing circuits 644, where each sensing circuit is coupled to one or more leads/electrodes and controlled by the microcontroller 620 to sense electrical activity detected at the corresponding one or more electrodes. The sensing circuit 624 may operate in, for example, a unipolar sensing configuration or a bipolar sensing configuration.

The IMD 600 further includes an analog-to-digital (A/D) data acquisition system (DAS) 650 coupled to one or more electrodes via the switch 626 to sample cardiac signals across any pair of desired electrodes. The A/D converter 650 is configured to acquire intracardiac electrogram signals, convert the raw analog data into digital data and store the digital data for later processing and/or telemetric transmission to an external device 690 (e.g., a programmer, local transceiver, or a diagnostic system analyzer). The A/D converter 650 is controlled by a control signal 656 from the microcontroller 620.

The microcontroller 620 is operably coupled to a memory 660 by a suitable data/address bus 662. The programmable operating parameters used by the microcontroller 620 are stored in the memory 660 and used to customize the operation of the IMD 600 to suit the needs of a particular patient. The operating parameters of the IMD 600 may be non-invasively programmed into the memory 660 through a telemetry circuit 664 in telemetric communication via communication link 667 (e.g., MICS, Bluetooth low energy, and/or the like) with the external device 690.

The IMD 600 can further include one or more physiological sensors 670. Such sensors are commonly referred to as "rate-responsive" sensors because they are typically used to adjust pacing stimulation rates according to the exercise state of the patient. However, the physiological sensor 670 may further be used to detect changes in cardiac output, changes in the physiological condition of the heart, or diurnal changes in activity (e.g., detecting sleep and wake states). Signals generated by the physiological sensors 670 are passed to the microcontroller 620 for analysis. While shown as being included within the IMD 600, the physiological sensor(s) 670 may be external to the IMD 600, yet still, be implanted within or carried by the patient. Examples of physiological sensors might include sensors that, for example, sense respiration rate, pH of blood, ventricular gradient, activity, position/posture, minute ventilation, and/or the like.

A battery 672 provides operating power to all of the components in the IMD 600. The battery 672 is capable of operating at low current drains for extended periods of time, and is capable of providing a high-current pulses (for capacitor charging) when the patient requires a shock pulse (e.g., in excess of 2 A, at voltages above 2 V, for periods of 10 seconds or more). The battery 672 also desirably has a predictable discharge characteristic so that elective replacement time can be detected. As one example, the IMD 600 employs lithium/silver vanadium oxide batteries.

The IMD 600 further includes an impedance measuring circuit 674, which can be used for many things, including sensing respiration phase. The impedance measuring circuit 674 is coupled to the switch 626 so that any desired electrode and/or terminal may be used to measure impedance in connection with monitoring respiration phase. The IMD 600 is further equipped with a communication modem (modulator/demodulator) 640 to enable wireless communication with other devices, implanted devices and/or external devices. In one implementation, the communication modem 640 may use high frequency modulation of a signal transmitted between a pair of electrodes. As one example, the signals may be transmitted in a high frequency range of approximately 10-80 kHz, as such signals travel through the body tissue and fluids without stimulating the heart or being felt by the patient.

Optionally, the microcontroller 620 may control a shocking circuit 680 by way of a timing control 632. The shocking circuit 680 generates shocking pulses as controlled by the microcontroller 620. The shocking circuit 680 may be controlled by the microcontroller 620 by a control signal 682.

Although not shown, the microcontroller 620 may further include other dedicated circuitry and/or firmware/software components that assist in monitoring various conditions of the patient's heart and managing pacing therapies. The microcontroller 620 further includes a timing control 632, an arrhythmia detector 634, a morphology detector 636 and multi-phase therapy controller 633. The timing control 632 is used to control various timing parameters, such as stimulation pulses (e.g., pacing rate, atria-ventricular (AV) delay, atrial interconduction (A-A) delay, ventricular interconduction (V-V) delay, etc.) as well as to keep track of the timing of RR-intervals, refractory periods, blanking intervals, noise detection windows, evoked response windows, alert intervals, marker channel timing, and the like.

The morphology detector 636 is configured to review and analyze one or more features of the morphology of cardiac activity signals. For example, in accordance with embodiments herein, the morphology detector 636 may analyze the morphology of detected R waves, where such morphology is then utilized to determine whether to include or exclude one or more beats from further analysis. For example, the morphology detector 636 may be utilized to identify non-conducted ventricular events, such as ventricular fibrillation and the like.

The arrhythmia detector 634 may be configured to apply one or more arrhythmia detection algorithms for detecting arrhythmia conditions. By way of example, the arrhythmia detector 634 may apply various detection algorithms. The arrhythmia detector 634 may be configured to declare a ventricular fibrillation episode based on the cardiac events.

The therapy controller 633 is configured to perform the operations described herein. The therapy controller 633 is configured to identify a multi-phase therapy based on the ventricular fibrillation episode, the multi-phase therapy including a pacing therapy. The therapy controller 633 is configured to manage delivery of the burst pacing therapy at a pacing site in a coordinated manner after the one or more shocks. The pacing site may be located at a target SOI, such as a His Bundle. Optionally, other pacing sites may be located at one of a left ventricular (LV) site or a right ventricular (RV) site. The therapy controller 633 may manage delivery of the shock along a shocking vector between shocking electrodes.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

The term "sized" as used herein is not limited to the act of manufacturing, but rather refers to a dimension similar to length, width, volume, etc. A lumen being sized to accommodate a specific component is not a method operation, but rather a characteristic of the lumen. The term "approximately" as used herein includes a threshold range of values above and below the stated value. For example, the threshold range may be +/- 5%, 3%, 2%, or the like.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the inventive subject matter without departing from its scope. While the dimensions and types of materials described herein are intended to define the parameters of the inventive subject matter, they are by no means limiting and are exemplary embodiments. Many other embodiments will be apparent to one of ordinary skill in the art upon reviewing the above description. The scope of the inventive subject matter should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A delivery system (100) comprising:
an access introducer sheath (102) that has a proximal end (120) and a distal end (122), the access introducer sheath (102) defining a channel (124) through a body (126) of the access introducer sheath (102) from the proximal end (120) to the distal end (122); and
a delivery catheter (103) disposed within the channel (124) of the access introducer sheath (102), the delivery catheter (103) defining a lumen (134) that extends along a central axis (114) of the delivery catheter (103), wherein a distal end segment (136) of the delivery catheter (103) projects beyond the distal end (122) of the access introducer sheath (102),
wherein the lumen (134) is configured to receive a dilator (106) therein that projects beyond a distal end (112) of the delivery catheter (103) so that the distal end segment (136) of the delivery catheter (103) is disposed between a tip segment (116) of the dilator (106) and the distal end (122) of the access introducer sheath (102) along the central axis (114).

2. The delivery system of claim 1, wherein the distal end (112) of the delivery catheter (103) is tapered.

3. The delivery system of claim 1 or 2, wherein the distal end (122) of the access introducer sheath (102) is tapered.

4. The delivery system of any one of claims 1 to 3, wherein the delivery catheter (103) has a step (150) that transitions from a broad segment (152) of the delivery catheter (103) to a narrow segment (154) of the delivery catheter (103), the broad segment (152) located between the distal end (112) of the delivery catheter (103) and the narrow segment (154),
wherein the access introducer sheath (102) surrounds the narrow segment (154) and the distal end (122) of the access introducer sheath (102) abuts against the step (150).

5. The delivery system of any one of claims 1 to 4, further comprising a hub (108) coupled to the access introducer sheath (102), the hub (108) comprising a housing (160) and a hemostasis seal (162).

6. The delivery system of claim 5, wherein the hub (108) is coupled to the proximal end (120) of the access introducer sheath (102) and the hemostasis seal (162) engages an outer surface (158) of the delivery catheter (103) at a location spaced apart from the access introducer sheath (102) to create a seal between the hub (108) and the delivery catheter (103).

7. The delivery system of claim 5 or 6, wherein the hemostasis seal (162) is an O-ring.

8. The delivery system of any one of claims 5 to 7, wherein the housing (160) of the hub (108) includes a base section (166) that has a flat back side (168) configured to be secured to skin of a patient to hold the access introducer sheath (102) in a fixed position on the patient.

9. The delivery system of claim 8, wherein the base section (166) includes an adhesive on the flat back side (168) for bonding to the skin of the patient.

10. The delivery system of any one of claims 1 to 9, wherein a length of the access introducer sheath (102) from the proximal end (120) to the distal end (122) is less than 10 cm.

11. The delivery system of any one of claims 1 to 10, wherein the access introducer sheath (102) has a wall thickness of no greater than 0.254 mm.

12. The delivery system of any one of claims 1 to 11, wherein the access introducer sheath (102) has a mono-material composition.

13. A method of assembling a delivery system (100), the method comprising:
inserting a delivery catheter (103) through a channel (124) of an access introducer sheath (102), wherein the channel (124) of the access introducer sheath (102) extends from a proximal end (120) of the access introducer sheath (102) to a distal end (122) of the access introducer sheath (102), wherein a distal end segment (136) of the delivery catheter (103) projects beyond the distal end (122) of the access introducer sheath (102); and
loading a dilator (106) through a lumen (134) of the delivery catheter (103) so that a tip segment (116) of the dilator (106) projects beyond the distal end segment (136) of the delivery catheter (103), wherein the distal end segment (136) of the delivery catheter (103) is disposed between the tip segment (116) of the dilator (106) and the distal end (122) of the access introducer sheath (102) along a central axis (114) of the delivery catheter (103).

14. The method of claim 13, further comprising:
coupling the access introducer sheath (102) to a hub (108) that comprises a housing (160) and a hemostasis seal (162); and
sealing the hub (108) to the delivery catheter (103) via the hemostasis seal (162) surrounding and engaging an outer surface (158) of the delivery catheter (103) at a location spaced apart from the access introducer sheath (102).

15. The method of claim 13 or 14, further comprising forming the access introducer sheath (102) to have at least one of: (i) a mono-material composition; (ii) a wall thickness of no greater than 0.254 mm; or (iii) a length that is less than 10 cm.
